Europäisches Patentamt

European Patent Office

Office européen des brevets

(11) Publication number: **0 224 504 B1**

(12) # EUROPEAN PATENT SPECIFICATION

(45) Date of publication of patent specification: **18.03.92**   (51) Int. Cl.⁵: **A61K 31/07**

(21) Application number: **86902974.4**

(22) Date of filing: **17.04.86**

(86) International application number:
**PCT/US86/00780**

(87) International publication number:
**WO 86/06275 (06.11.86 86/24)**

(54) **COMPOSITION AND METHOD FOR REDUCING WRINKLES.**

(30) Priority: **22.04.85 US 725480**
           **28.03.86 US 845256**

(43) Date of publication of application:
**10.06.87 Bulletin 87/24**

(45) Publication of the grant of the patent:
**18.03.92 Bulletin 92/12**

(84) Designated Contracting States:
**DE FR GB IT**

(56) References cited:
**EP-A- 0 094 771        WO-A-85/03434**
**GB-A- 2 065 687        GB-A- 2 155 337**
**US-A- 4 224 319        US-A- 4 423 041**
**US-A- 4 425 364**

**L. KLIGMAN, "The effect on Rhino Mouse Skin of Agents Which Influence Keratinization and Exfoliation" The Journal of Investigative Dermatology, Vol. 73, No. 5, Part 1, pages 354 to 358, see the entire document. Pub. Williams & Wilkins Co., Baltimore, Mary-**

**land, U.S.A.**

(73) Proprietor: **AVON PRODUCTS, INC.**
**9 West 57th Street**
**New York, NY 10019(US)**

(72) Inventor: **WILMOTT, James, M.**
**137 Rolling Ridge Road**
**West Milford, NJ 07480(US)**
Inventor: **ZNAIDEN, Alexander, P.**
**155 Johnstown Road**
**Sloatsburgh, NY 10947(US)**

(74) Representative: **Holmes, Michael John et al**
**Frank B. Dehn & Co. Imperial House 15-19**
**Kingsway**
**London WC2B 6UZ(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

Background of the Invention

a) Field of the Invention

This invention relates to stable compositions containing retinol which upon topical application to the skin cause wrinkle effacement and other beneficial effects. It also relates to use of such compositions in combination with or including moisturizers.

b) State of the Art

Retinol (Vitamin A), though believed to cause beneficial skin effects, has never been successfully formulated in a composition suitable for topical application. It has now been discovered that retinol can be formulated in stable form and can be administered topically in a cosmetic base with minimal irritant side effects. Further, when topically applied as taught by the invention, retinol causes effacement of facial fine lines and wrinkles, increases skin elasticity, reduces pore size and improves skin texture.

Summary of the Invention

This invention relates to stable cosmetic compositions for reducing facial lines and wrinkles and otherwise enhancing skin quality and to methods of using that composition. The compositions comprise retinol coupled with a volatile silicone carrier by means of a mutual solvent. The preferred compositions, which result in optimum stability and efficacy, are those containing volatile silicones, such as cyclomethicone, ethanol as the mutual solvent and retinol. When applied to the skin, the compositions contain 0.005 to 1.0 weight percent retinol. Most preferred compositions for application to the skin contain 0.01 to 0.50 weight percent retinol. The stable compositions of the invention may be formulated with more concentrated levels of retinol and may be diluted to suitable levels of retinol for application to the skin by means of cosmetically acceptable carriers. The compositions may additionally contain or may be applied in conjunction with a moisturizer to enhance the beneficial effects and sensory comfort of the composition.

Detailed Description of the Invention

It has been discovered that a cosmetic composition containing retinol in a volatile silicone vehicle is stable, and upon topical application reduces fine lines and wrinkles and otherwise beneficially affects skin quality. More particularly, it has been discovered that a stable retinol composition can be formulated using a volatile silicone carrier and a mutual solvent for the silicone and retinol. Compositions containing 0.005 to 1.0 weight percent retinol can reduce facial fine lines and wrinkles with minimal irritant effects.

The stable retinol compositions of the invention are formulated in a vehicle containing a volatile silicone. With such a vehicle, retinol levels needed to achieve beneficial effects are minimized and the potential for irritant effects to the skin by retinol are greatly diminished. Moreover, retinol is stable when formulated in the silicone containing compositions of the invention, in contrast to other conventional cosmetic carriers.

The compositions of the invention may be formulated as alcohol lotions containing 0.005 to 1.0 weight percent retinol, in which case they may be applied directly to the skin, or as more concentrated alcohol lotions containing higher levels of retinol, in which case prior to application they are diluted by means of a cosmetically acceptable carrier to a 0.005 to 1.0 weight percent retinol level. In the formulations of the invention, water is optimally minimized or eliminated to maximize the stability of retinol and to minimize the potential for separation of the oil and water. No more than 2 percent water should be present.

When the compositions of the invention are formulated for application as alcohol lotions, a preferred mutual solvent for the volatile silicone and the retinol is ethanol. Preferred silicones are volatile low viscosity polysiloxanes, such as cyclomethicones, including cyclomethicone tetramer and pentamer. In the alcohol lotion formulation, the retinol level is most preferably 0.10 to 0.50 weight percent. The weight ratio of silicone to mutual solvent is optimally about 6:4.5 when cyclomethicone tetramer is the silicone and ethanol is the mutual solvent. At that level, stability, comfort and aesthetics are optimized. For stability the silicone should be at least 20 weight percent of the alcohol lotion composition. The ethanol or other mutual solvent, such as isopropyl alcohol, should be present in an amount sufficient to couple the silicone and retinol and yet not exceed the level at which tearing, stinging or other discomfort is observed. Generally, levels of ethanol between 25 to 60 weight percent will be acceptable.

An alcohol lotion composition may additionally contain emollients, and thickening agents for aesthetic effects such as lubrication and tactile perception. The emollients which may be present include all standard emollients which are miscible in the alcohol lotion. Preferred emollients include ethyl hexyl palmitate, neopentyl glycol dicaprate and ethylene glycol dicaprate. The quantity thereof is dependent upon tactile perception and compatibility with the silicone and solvent. Levels up to 15 weight percent will commonly be acceptable.

Ultraviolet absorbers or sunscreens, antioxidants and the like may also be present in the compositions of the invention to enhance the stability of retinol against degradation. Examples of ultraviolet absorbers which may be employed in the alcohol lotion formulation include octyl dimethyl PABA and benzophenone-3. Examples of suitable antioxidants and preservatives include butylated hydroxytoluene, BHA, imidazoline urea and methylparaben. Thickeners are those which are compatible with the overall composition, such as bentones, fumed silica and ethyl cellulose. Dyes, fragrance and other cosmetic additives may also be present, if desired, provided they do not react with any component of the composition and do not interfere with the homogeneity of the composition. The stable retinol/silicone compositions of the invention may be formulated in concentrated form, that is, may be formulated with higher levels of retinol, without loss of stability. However, when so formulated the compositions of the invention are diluted to produce retinol levels of 0.005 to 1.0 weight percent, preferably 0.01 to 0.50 weight percent, prior to application to the skin. In preferred practice, dilution may be effected by means of a water in oil emulsion. In such an emulsion, silicones of the type employed in the alcohol lotion are preferred. The silicone will commonly be 15 to 35 weight percent of the composition which is applied to the skin and most preferably 20 weight percent of the composition.

A suitable emulsion for dilution of the compositions of the invention may be formed by first mixing the volatile silicones with silicone dimethicone copolyol. The remaining components of the emulsion, such as emulsifiers, emulsion stabilizers, preservatives and sunscreens, are dissolved or dispersed in water. The aqueous phase is then very slowly added to the cyclomethicone phase with vigorous homogenization which is continued until an acceptable viscosity is achieved.

The aqueous phase of the emulsion should contain preservatives of the type indicated for the alcohol lotion. It is also desirable that emulsion stabilizers, such as sodium chloride be employed.

The relative ratios of the water and oil phases is optimally 2 to 1. Deviations from this ratio result in changes in the viscosity of the system. The limits of permissible variation are dependent upon cosmetically acceptable viscosities.

When the composition of the invention is formulated in concentrated form for application in an emulsion of the type described, the emulsion and composition are blended prior to application. The quantity of emulsion employed is dependent on the concentration of the retinol composition of the invention. For example, if the retinol composition contains 4 weight percent retinol, sufficient emulsion must be added to reduce the percent weight of retinol to the 0.005 to 1.0 range, or more preferably to the 0.01 to 0.50 range.

The compositions of the invention, when formulated in concentrated form, can also be diluted to the appropriate retinol level for application by means of other cosmetically acceptable carriers or vehicles which are miscible with the retinol composition of the invention. Other cosmetic additives may be employed, either in the compositions of the invention or in those compositions when diluted with a suitable vehicle, provided they do not react with the retinol and are otherwise compatible with the compositions.

The compositions formulated as described above are topically applied to the skin on concentration which result in application of 0.005 to 1.0 weight percent retinol, preferably 0.01 to 0.50 weight percent. They are applied in the areas where fine lines, wrinkles, dry or inelastic skin or large pores are observed. Optimally a moisturizer is applied with or after application of the retinol compositions to enhance the tactile comfort associated with application of the compositions and to enhance the wrinkle effacement and other benefits achieved by the compositions.

Alternatively, it has been discovered that moisturizing efficacy can be achieved in the compositions of the present invention containing the retinol, thereby precluding the need for a separate moisturizer. Therefore, preferred compositions of the invention can be formulated to include moisturizing components that are compatible with the alcohol lotion or silicone emulsion to a level of up to 35% by weight of the final formulation. Preferred moisturizing ingredients suitable for use the preferred compositions of the invention may be selected from gly acrylic polymer (Lubragel®), petrolatum, ethylhexyl palmitate, and hyaluronic acid sodium salt.

With daily application, skin texture, color and tone will improve. Wrinkles and fine lines will be reduced with minimal irritant effects.

The following examples are illustrative of the invention and of the beneficial effects which can be achieved with the compositions of the invention.

Example 1

A preferred alcohol lotion composition in accordance with the invention was formulated as follows (all quantities are stated as weight percents):

A retinoid blend was formulated containing:

| | |
|---|---|
| 48.01264 | Polysorbate 20 |
| 48.01264 | Retinol |
| 3.00000 | BHT |
| .75000 | BHA |
| .09977 | Retinol Acetate |
| .09977 | Retinol Palmitate |
| .02494 | Carotene |
| .00024 | Apocarotenal |

The blend was combined with an alcohol lotion composition to form the following composition of the invention:

```
            Formulation A

    46.27776%          Cyclomethicone-Tetramer
    35.00000%          Alcohol SD 40B Anhydrous
     5.00000%          Ethylhexyl Palmitate
     5.00000%          Octyl Dimethyl PABA
     2.00000%          Benzophenone-3
     2.00000%          Demineralized Water
     2.00000%          Neopentyl Glycol Dicaprate
     1.50000%          Ethyl Cellulose K5000
     0.22000%          Butylated Hydroxytoluene
     1.00224%          Retinoid Blend

Total   100.00000%
```

Example 2

A preferred emulsion was formulated as follows. A concentrated retinoid composition in accordance with the invention was formulated as follows:

| | |
|---|---|
| Retinoid Blend (Example 1) | 4.17600 |
| BHT | .16660 |
| Alcohol SD40B Anyhydrous | 33.33330 |
| Cyclomethicone-Tetramer | 62.32410 |

A water in oil emulsion was formulated as follows:

| Cyclomethicone Tetramer | 11.36360 |
| Cyclomethicone Pentamer | 5.68180 |
| Cyclomethicone/Dimethicone Copolyol | 11.36360 |
| Demineralized Water | 64.91370 |
| Sodium Chloride | 1.13640 |
| Methylparaben | .45450 |
| Stearyl ETO (20M) Alcohol | 1.13640 |
| Triethanolamine 99% | 1.25000 |
| Phenyl Benzimidazole | 2.27270 |
| BHT | .22730 |
| Germall® (Sutton Laboratories) | .20000 |

Before application, the concentrated retinoid composition and the emulsion are blended to form the following composition suitable for application to the skin.

## Formulation B

| | |
|---|---|
| 57.12406% | Demineralized Water |
| 16.97770% | Cyclomethicone-Tetramer |
| 10.00000% | Cyclometh/dimeth Copolyol 90/10 |
| 5.00000% | Cyclomethicone-Pentamer |
| 4.00000% | Alcohol SD 40B Anhydrous |
| 2.00000% | Phenyl Benzimidazole-5-Sulf Acid |
| 1.10000% | Triethanolamine 99% |
| 1.00000% | Stearyl ETO (20M) Alcohol |
| 1.00000% | Sodium Chloride |
| 0.40000% | Methylparaben |
| 0.22000% | Butylated Hydroxytoluene |
| 1.00224% | Retinoid Blend |
| 0.17600% | Germall® |

Total     100.00000%

Example 3

A commercially available Vitamin A Alcohol Blend (Retinol Blend) was obtained comprising the following ingredients by percent weight:

| | |
|---|---|
| Polysorbate 20 | 48.125 |
| Retinol | 48.125 |
| BHT | 3.000 |
| BHA | 0.750 |
| | 100.000% |

This produced a composition which contained approximately 1.5 million units of Vitamin A activity per gram. This Retinol Blend or composition was used to prepare the following test formulations:

|  | Alcohol Lotion | Emulsion |
|---|---|---|
| Alcohol SD 40B Anhydrous | 35.00000 | 4.00000 |
| Benzophenone-3 | 2.00000 |  |
| Neopentyl Glycol Dicaprate | 2.00000 |  |
| Demineralized Water | 2.00000 | 59.50000 |
| Ethylcellulose K5000 | 1.50000 |  |
| Cyclomethicone-Tetramer | 45.50000 | 15.00000 |
| Ethyhexyl Palmitate | 5.00000 |  |
| Octyl Dimethyl PABA | 5.00000 |  |
| Vitamin A Alcohol Blend | 1.00000 | 1.00000 |
| Fumed Silica | 1.00000 |  |
| Cyclomethicone Pentamer |  | 5.00000 |
| Cyclomethicone Dimethicone Copolyol |  | 10.00000 |
| Sodium Chloride |  | 1.00000 |
| Methylparaben |  | .40000 |
| Stearyl ETO (20M) Alcohol |  | 1.00000 |
| Triethanolamine 99% |  | 1.10000 |
| Phenyl Benzamidazole |  | 2.00000 |

A split face test was conducted by using the foregoing following formulations as follows. Twelve females aged 20 to 59 applied one of the test formulations to one side of their faces and the other to the other side once daily for eight weeks. Rich Moisture Cream (Avon), a moisturizer, was applied over both retinol treated areas after application of the test formulation. Thin shavings of the skin on each side of the face were taken before the test began and after the eight week test period. It was observed that the skin shavings after the test were in better condition than those before the test in nine of the twelve women. The skin of those nine women was both thicker and more organized after the test than before. There were no observable differences between the effects of the two test formulations.

Example 4

Rhino mouse skin studies were conducted to determine the effectiveness of retinol in normalizing epidermal skin structures. Rhino mice normally have wrinkled, sagging skin. The rhino mouse test is used as a model for showing the effects of compositions on the epidermis. In the test each set of seven mice was treated for five days/week for six consecutive weeks. Eight sets of mice were treated. The treating agents were similar to the vehicles of Formulations A and B in Examples 1 and 2 without the retinol blend and each of those vehicles with the retinol blend, with the retinol content at concentrations of 0.10, 0.25 and 0.50 weight percent. Specifically, the four silicone emulsions contained:

|  | I | II | III | IV |
|---|---|---|---|---|
| Cyclomethicone-Pentamer | 5.00000 | 5.00000 | 5.00000 | 5.00000 |
| Cyclomethicone-Tetramer | 17.50000 | 16.97300 | 16.97300 | 16.97300 |
| Cyclometh/Dimeth Copolyol 90 | 10.00000 | 10.00000 | 10.00000 | 10.00000 |
| Demineralized Water | 59.50000 | 59.50000 | 59.50000 | 59.50000 |
| Sodium Chloride | 1.00000 | 1.00000 | 1.00000 | 1.00000 |
| Methylparaben | .40000 | .40000 | .40000 | .40000 |
| Stearyl ETO (20M) Alcohol | 1.00000 | 1.00000 | 1.00000 | 1.00000 |
| Triethanolamine 99% | 1.10000 | 1.10000 | 1.10000 | 1.10000 |
| Phenyl Benzimidazole-5-Sulf. | 2.00000 | 2.00000 | 2.00000 | 2.00000 |
| Alcohol SD 40B Anhydrous | 2.50000 | 2.80000 | 2.50000 | 2.00000 |
| Vitamin A Alcohol Blend | -------- | .20000 | .50000 | 1.00000 |
| Carotenoid Solution | -------- | .02500 | .02500 | .02500 |
| Vitamin A Palmitate | -------- | .00100 | .00100 | .00100 |
| Vitamin A Acetate | -------- | .00100 | .00100 | .00100 |

The four alcohol lotion formulations contained:

| | V | VI | VII | VIII |
|---|---|---|---|---|
| Alcohol SD 40B Anhydrous | 35.00000 | 35.30000 | 35.00000 | 34.50000 |
| Ethylhexyl Palmitate | 5.00000 | 5.00000 | 5.00000 | 5.00000 |
| Benzophenone-3 | 2.00000 | 2.00000 | 2.00000 | 2.00000 |
| Octyl Dimethyl PABA | 5.00000 | 5.00000 | 5.00000 | 5.00000 |
| Neopentyl Glycol Dicaprate | 2.00000 | 2.00000 | 2.00000 | 2.00000 |
| Demineralized Water | 2.00000 | 2.00000 | 2.00000 | 2.00000 |
| Ethyl Cellulose K5000 | 1.50000 | 1.50000 | 1.50000 | 1.50000 |
| Butylated Hydroxytoluene | .20000 | .22000 | .22000 | .22000 |
| Cyclomethicone-Tetramer | 46.30000 | 45.75300 | 45.75300 | 45.75300 |
| Silica-Fumed | 1.00000 | 1.00000 | 1.00000 | 1.00000 |
| Carotenoid Solution | -------- | .02500 | .02500 | .02500 |
| Vitamin A Acetate | -------- | .00100 | .00100 | .00100 |
| Vitamin A Palmitate | -------- | .00100 | .00100 | .00100 |
| Vitamin A Alcohol Blend | -------- | .20000 | .50000 | 1.00000 |

Visual observation of the skin condition during the period of treatment showed a diminution of the characteristic epidermal glandular structures in the sets of mice treated with formulations containing retinol. No such change in wrinkling and sagging was observed in the mice treated with the vehicle alone.

Example 5

A test of the effectiveness of retinol formulations of the invention in reducing damage to skin resulting from exposure to sunlight, and more particularly ultraviolet wavelengths thereof, was conducted. The test was conducted using hairless mice. Sixty mice were exposed three days a week for ten weeks to 20 minutes of ultraviolet light. Thereafter the mice were divided into four groups of 5 mice each. Each group was treated with one of the four following compositions:

| Emulsion | | |
|---|---|---|
| Cyclomethicone Pentamer | 5.00000 | 5.00000 |
| Cyclomethicone Tetramer | 16.97300 | 17.50000 |
| Cyclometh/Dimeth Copolyol | 10.00000 | 10.00000 |
| Demineralized Water | 59.50000 | 59.50000 |
| Sodium Chloride | 1.00000 | 1.00000 |
| Methylparaben | .40000 | .40000 |
| Stearyl ETO (20M) Alcohol | 1.00000 | 1.00000 |
| Triethanolamine 99% | 1.10000 | 1.10000 |
| Phenyl Benzimidazole | 2.00000 | 2.00000 |
| Alcohol SD 40B Anhydrous | 2.00000 | 2.50000 |
| Vitamin A Alcohol Blend | 1.00000 | |
| Carotinoid Solution | .02500 | |
| Vitamin A Palmitate | .00100 | |
| Vitamin A Acetate | .00100 | |

| Alcohol Lotion | | |
|---|---|---|
| Alcohol SD 40B Anhydrous | 34.50000 | 35.00000 |
| Ethylhexyl Palmitate | 5.00000 | 5.00000 |
| Benzophenone-3 | 2.00000 | 2.00000 |
| Octyl Dimethyl PABA | 5.00000 | 5.00000 |
| Neopentyl Glycol Dicaprate | 2.00000 | 2.00000 |
| Demineralized Water | 2.00000 | 2.00000 |
| Ethyl Cellulose K5000 | 1.50000 | 1.50000 |
| BHT | .22000 | .20000 |
| Cyclomethicone Tetramer | 45.75300 | 46.30000 |
| Fumed Silica | 1.00000 | 1.00000 |
| Carotinoid Solution | .02500 | |
| Vitamin A Acetate | .00100 | |
| Vitamin A Palmitate | .00100 | |
| Vitamin A Alcohol Blend | 1.00000 | |

Treatment was effected daily for five weeks. Another four groups of seven mice each were similarly treated for ten weeks.

After treatment, the skin repair of the UV-induced damage was observed. The skin of mice treated with retinol-containing formulations had up to a twofold greater repair zone than the skin of mice treated with either vehicle. This greater repair can be correlated with visual improvement in the skin. That is, under similar treatment with retinol, the human skin can be expected to show a diminution in lines and wrinkles.

Example 6

A six month study was conducted to determine the ability of retinol to produce visible effacement of facial crowsfeet, lines and wrinkles and textural skin improvements. The test was done on a split face basis with half of the test population applying Formulation C and the other half using Formulation D:

| | C | D |
|---|---|---|
| Cyclomethicone-Tetramer | 15.00000 | 45.30000 |
| Cyclomethicone-Pentamer | 5.00000 | -------- |
| Cyclomethy/Dimeth Copolyol 90/10 | 10.00000 | -------- |
| Demineralized Water | 59.50000 | 2.00000 |
| Sodium Chloride | 1.00000 | -------- |
| Methylparaben | .40000 | -------- |
| Stearyl ETO (20M) Alcohol | 1.00000 | -------- |
| Triethanolamine 99% | 1.10000 | -------- |
| Phenyl Benzimidazole-5-Sulf Acid | 2.00000 | -------- |
| Alcohol SD 40B Anhydrous | 4.00000 | 35.00000 |
| Vitamin A Alcohol Blend | 1.00000 | 1.00000 |
| Butylated Hydroxytoluene | -------- | .20000 |
| Ethylhexyl Palmitate | -------- | 5.00000 |
| Benzophenone-3 | -------- | 2.00000 |
| Octyl Dimethyl PABA | -------- | 5.00000 |
| Neopentyl Glycol Dicaprate | -------- | 2.00000 |
| Ethyl Cellulose K5000 | -------- | 1.50000 |
| Silica-Fumed | -------- | 1.00000 |

Crowsfeet, suborbital areas, cheeks and lips were treated. Application was done once a day.

Within two months of commencement of the test, the benefits of the retinol formulations were evident. Textural skin improvement was observed in the crowsfeet and cheek areas of the panelists. A softening or smoothing in fine lines in the crowsfeet and suborbital areas was also noted.

At the end of three months, line reduction in the crowsfeet and suborbital area appeared in more panelists than at the end of two month period. There was also a greater improvement in skin texture in the crowsfeet and cheek areas between the 2 and 3-month observations. Further, textural improvement in the suborbit of the eye and decreased dryness in the crowsfeet, suborbital and cheek areas were apparent.

After four months of treatment, the skin areas treated with the formulations of the invention showed marked improvement. Skin dryness and texture had improved in all four treated areas. Lines and wrinkles had diminished in the crowsfeet suborbital and cheek areas. The skin in the crowsfeet and suborbital areas also showed a significant improvement in firmness.

By the end of six months of treatment, the benefits of the formulations of the invention were fully apparent. With the exception of the lip area, there was a visual improvement in the treated skin's texture and the treated skin was significantly smoother to the touch. There had also been a significant reduction in the number of fine lines and/or wrinkles in the crowsfeet and suborbital regions and to a lesser extent on the cheeks.

Example 7

A water in oil emulsion in accordance with the invention was formulated as follows:

|  | % |
| --- | --- |
| Volatile Silicone Tetramer | 15.0 |
| Volatile Silicone Pentamer | 5.0 |
| Dimethicone Copolyol | 10.0 |
| Water | q.s. |
| Eusolex 232® | 2.0 |
| TEA 99% | 1.1 |
| Sodium Chloride | 1.0 |
| Ethanol | 4.0 |
| Retinoid Blend 1.5MM units (Example 1) | 1.0 |
| Bridge 78 (20) ethoxylated Stearyl Ether | 1.0 |

This product possesses excellent aesthetic properties and the retinol is acceptably stable in this vehicle over the intended usage period. Its efficacy has been established via the Rhino mouse test described in Example 4.

Example 8

The stability of retinol in the compositions of the invention was tested. The formulations tested were as follows:

## Formulation C

| | |
|---|---:|
| Cyclomethicone-Tetramer | 58.1481 |
| Anhydrous Alcohol | 33.3333 |
| Retinoid Blend | 8.3520 |
| BHT | 0.1666 |
| | 100.0000% |

## Formulation D

| | |
|---|---:|
| Cyclomethicone-Tetramer | 45.500 |
| Anhydrous Alcohol | 35.000 |
| Ethylhexyl Palmitate | 5.000 |
| Octyl Dimethyl PABA | 5.000 |
| Benzophenone-3 | 2.000 |
| Neopentyl Dicaprate | 2.000 |
| Demineralized Water | 2.000 |
| ECK-5000 | 1.500 |
| Retinol Blend | 1.000 |
| Fumed Silica | 1.000 |
| | 100.000% |

### Formulation E

| | |
|---|---:|
| Cyclomethicone-Tetramer | 39.80 |
| Anhydrous Alcohol | 40.00 |
| Retinoid Blend | 10.00 |
| BHT | 0.20 |
| | 100.00 |

### Formulation F

| | |
|---|---:|
| Cyclomethicone-Tetramer | 69.53 |
| Anhydrous Alcohol | 20.00 |
| Retinol Blend | 10.00 |
| BHT | 0.20 |
| Carotinoid-Solution | 0.25 |
| Retinyl Palmitate | 0.01 |
| Retinyl Acetate | 0.01 |
| | 100.00% |

### Formulation G

| | |
|---|---:|
| Cyclomethicone-Tetramer | 34.765 |
| Ethylhexyl Palmitate | 25.000 |
| Octyl Dimethyl PABA | 25.000 |
| Anhydrous Alcohol | 10.000 |
| Retinol Blend | 5.000 |
| Carotinoid Solution | 0.125 |
| BHT | 0.100 |
| Retinyl Palmitate | 0.005 |
| Retinyl Acetate | 0.005 |
| | 100.000% |

### Formulation H

| | |
|---|---:|
| Anhydrous Alcohol | 56.00 |
| Cyclomethicone-Tetramer | 22.00 |
| Octyl Dimethyl PABA | 8.00 |
| Ethylhexyl Palmitate | 5.00 |
| Benzophenone-3 | 3.00 |
| Neopentyl Dicaprate | 2.00 |
| Demineralized Water | 2.00 |
| Hydroxypropyl Cellulose | 1.30 |
| Retinol Blend | 0.50 |
| BHT | 0.20 |
| | 100.00% |

### Formulation I

| | |
|---|---:|
| Anhydrous Alcohol | 55.50 |
| Cyclomethicone-Tetramer | 22.00 |
| Octyl Dimethyl PABA | 8.00 |
| Ethylhexyl Palmitate | 5.00 |
| Benzophenone-3 | 3.00 |
| Neopentyl Dicaprate | 2.00 |

```
Demineralized Water                    2.00
Hydroxypropyl Cellulose                1.30
Retinol Blend                          1.00
BHT                                    0.20
                                     100.00%
```

The retinol blend used in the foregoing formulations was the same as the Vitamin A Alcohol Blend of Example 3, namely:

```
Polysorbate 20                        48.125
Retinol                               48.125
BHT                                    3.000
BHA                                    0.750
                                     100.000%
```

The retinoid blend used in the foregoing formulations was as follows:

```
Polysorbate 20                        48.01264
Retinol                               48.01264
BHT                                    3.00000
BHA                                    0.75000
Retinyl Palmitate                      0.09977
Retinyl Acetate                        0.09977
Carotene                               0.02494
Apocarotenal                           0.00024
                                     100.00000%
```

The Carotinoid Solution was as follows:

```
Ethylhexyl Palmitate                  98.988
Beta-Carotene                          1.000
Canthaxanthine                         0.002
Apocarotenal                           0.010
                                     100.000%
```

The results were as follows:

| 100 o F | | | | | | | |
|---|---|---|---|---|---|---|---|
| Formulation | | | | | | | |
| Week | C | D | E | F | G | H | I |
| 0 | --- | --- | --- | --- | --- | --- | --- |
| 1 | --- | 92% | 99% | 94% | 103% | 104% | 102% |
| 2 | 99% | 88% | 98% | 97% | 103% | 110% | 98% |
| 3 | 102% | 81% | --- | --- | --- | --- | --- |
| 4 | 100% | --- | --- | 97% | 104% | 108% | 87% |
| 6 | 101% | 80% | 98% | 93% | 100% | --- | --- |
| 8 | 97% | 79% | --- | 89% | 95% | 94% | 95% |
| 12 | --- | 82% | 93% | 89% | 97% | 87% | 94% |

| Room Temperature | | | | | | | |
|---|---|---|---|---|---|---|---|
| Formulation | | | | | | | |
| Week | C | D | E | F | G | H | I |
| 0 | 100% | 100% | 100% | 100% | 100% | 100% | 100% |
| 1 | --- | --- | --- | --- | --- | 108% | 100% |
| 2 | --- | 101% | --- | --- | --- | 112% | 101% |
| 3 | 102% | 94% | --- | --- | --- | --- | --- |
| 4 | 101% | --- | --- | --- | --- | 104% | 99% |
| 6 | 100% | 98% | --- | --- | --- | --- | --- |
| 8 | 99% | 96% | --- | --- | --- | 106% | 104% |
| 12 | 99% | 87% | 98% | 96% | 101% | 102% | 99% |

Example 9

An alternatively preferred emulsion was formulated by forming a composition in accordance with the invention as follows:

| | |
|---|---|
| Retinoid Blend (Example 1) | 0.1740 |
| BHT | 0.1666 |
| Alcohol SD40B Anhydrous | 33.3333 |
| Cyclomethicone-Tetramer | 66.3238 |
| Beta-Carotene | 0.0023 |

A water-in-oil emulsion was formulated as follows:

| | |
|---|---|
| Cyclomethicone-Tetramer | 7.38560 |
| Cyclomethicone-Pentamer | 3.97800 |
| Cyclometh/Dimeth Copolyol 90/10 | 11.36360 |
| Petrolatum | 5.68180 |
| Ethylhexyl Palmitate | 5.68180 |
| Demineralized Water | 36.15350 |
| Sodium Chloride | 1.13640 |
| Methylparaben | 0.45450 |
| Stearyl ETO (20M) Alcohol | 1.13640 |
| Gly Acrylic Polymer (Lubragel) | 22.72730 |
| Hyaluronic Acid Sod. Salt | 0.05110 |
| Triethanolamine 99% | 1.55000 |
| Phenyl Benzimidazole-5-Sulf Acid | 2.27270 |
| Butylated Hydroxytoluene | 0.22730 |
| Germall | 0.20000 |

As in prior examples, before application, the concentrated retinol composition and the emulsion were blended together to form the following composition suitable for topical application to the skin.

## Formulation J

| | |
|---|---|
| Demineralized Water | 31.81508 |
| Gly Acrylic Polymer (Lubragel) | 20.00020 |
| Cyclomethicone-Tetramer | 14.45792 |
| Cyclometh/Dimeth Copolyol 90/10 | 10.00000 |
| Cyclomethicone-Pentamer | 3.50064 |
| Petrolatum | 5.00000 |
| Ethylhexyl Palmitate | 5.00000 |
| Alcohol SD 40B Anhydrous | 4.00000 |
| Phenyl Benzimidazole-5-Sulf Acid | 2.00000 |
| Triethanolamine 99% | 1.36400 |
| Stearyl ETO (20M) Alcohol | 1.00000 |
| Sodium Chloride | 1.00000 |
| Hyaluronic Acid Sod. Salt | 0.04500 |
| Methylparaben | 0.40000 |
| Butylated Hydroxytoluene | 0.22000 |
| Retinoid Blend (Example I) | 0.02088 |
| Germall® | 0.17600 |
| Beta-Carotene | 0.00028 |
| | 100.00000% |

It will be noted that this composition (Formulation J) and that of Example 10 below includes moisturizing ingredients such as gly acrylic polymer (Lubragel), petrolatum, ethylhexyl palmitate, and hyaluronic acid sodium salt.

### Example 10

A split face test was conducted using the following emulsion formulation:

| | |
|---|---|
| Demineralized Water | 32.07848 |
| Gly Acrylic Polymer (Lubragel) | 20.00020 |
| Cyclomethicone-Tetramer | 14.45852 |
| Cyclometh/Dimeth Copolyol 90/10 | 10.00000 |
| Cyclomethicone-Pentamer | 3.50064 |
| Petrolatum | 5.00000 |
| Ethylhexyl Palmitate | 5.00000 |
| Alcohol SD 40B Anhydrous | 4.00000 |
| Phenyl Benzimidazole-5-Sulf Acid | 2.00000 |
| Triethanolamine 99% | 1.10000 |
| Stearyl ETO (20M) Alcohol | 1.00000 |
| Sodium Chloride | 1.00000 |
| Hyaluronic Acid Sod. Salt | .04500 |
| Methylparaben | .40000 |
| Butylated Hydroxytoluene | .22000 |
| Retinoid Blend (Example I) | .02088 |
| Germall® | .17600 |
| Beta-Carotene | .00028 |

Sixteen females aged 30-54 applied the test formulation to one side of their face twice daily for six weeks, the other side remained untreated. A leading clinical dermatologist observed a significant reduction in the appearance of the signs of facial aging including: diminution of skin lines and an improvement in the look and feel (texture) of facial skin.

### Example 11

14

The panelists who participated in the study in Example 10 were instrumentally assessed for improvement in facial lines via digital image processing of skin surface replicas (Image Analysis) and by Optical Profilometry, a measure of the degree of skin surface smoothness. After 6 weeks of product usage, there was a 26% reduction in skin lines as measured by Image Analysis, and there was a 41% improvement in skin smoothness as shown by Optical Profilometry.

Example 12

The ability of an electric current to flow through the stratum corneum provides an indirect measurement of the corneum's water content. The panelists who participated in the study in Example 10 were assessed for moisturization using an IBS impedance/conductance meter. At least twelve hours elapsed between the last product application and the skin conductance measurement. The data clearly showed that the treated side was more moist (higher conductance readings at all measurement time points). Further, the untreated control side of the face decreased in relative moisture content while the test side increased. Thus, the objective measurement and substantiation of the stratum corneum's electrical conductivity showed a significant enhancement in facial skin moisture content.

Example 13

The effectiveness of Formulation J in Example 9 on Skin biomechanical properties was evaluated by the in-vivo extensometer. One forearm site of thirteen panelists and one leg site from 10 panelists were treated with the composition of Formulation J. The contralateral forearm or leg respectively served as the untreated control. Both single and multiple applications were measured for a usage period of up to seven days. The results from this study indicated a product induced change in the skin's biomechanical properties. There was a 36% improvement in skin extensibility/elasticity immediately after product application, a 26% improvement for up to three hours after multiple product application. All these values were statistically significant.

Example 14

A test of the ability of a retinol containing composition to enhance the rate of cellular turnover in the epidermis was conducted. The human epidermis represents a cell renewal system in which fully differentiated cells (corneocytes) are being continually shed from the skin surface. Since this system operates under steady-state conditions, this loss of desquamated cells must be balanced by new cell production in the germinative cell layers. One parameter that is especially important to measure in such a system is transit time - the time required for a cell to move through a compartment. Since cells move in unison as a layer through the stratum corneum,, this means, in this special case, that transit time is equivalent to turnover time - the time required for a compartment to completely renew itself.

Previous studies have shown that the turnover time of the stratum corneum can be measured nointrusively by impregnating it with a fluorescent marker dye that binds avidly to the nonviable corneocytes but not the underlying viable epidermal cells. Thus, the time required for the dye to disappear, which can be monitored by Wood's lamp examination, is an indication of the turnover time of the stratum corneum. Therefore,, any difference in the time required for the dye to disappear from a treated and a nontreated site can be considered to be an expression of that product's ability to enhance epidermal renewal.

A test of fifteen healthy female subjects aged 25-45 participated in the study. One arm site was untreated, the other was treated with a composition according to the present invention comprising:

15

| Demineralized Water | 32.08388 |
|---|---|
| Gly Acrylic Polymer (Lubragel)® | 20.00020 |
| Cyclomethicone-Tetramer | 14.45852 |
| Cyclometh/Dimeth Copolyol 90/10 | 10.00000 |
| Cyclomethicone-Pentamer | 3.50064 |
| Petrolatum | 5.00000 |
| Ethylhexyl Palmitate | 5.00000 |
| Alcohol SD 40B Anhydrous | 4.00000 |
| Phenyl Benzimidazole-5-Sulf Acid | 2.00000 |
| Triethanolamine 99% | 1.10000 |
| Stearyl ETO (20M) Alcohol | 1.00000 |
| Sodium Chloride | 1.00000 |
| Hyaluronic Acid Sod. Salt | .03960 |
| Methylparaben | .40000 |
| Butylated Hydroxytoluene | .22000 |
| Retinoid Blend (Example I) | .02088 |
| Germall® | .17600 |
| Beta-Carotene | .00028 |

A two week pretreatment was conducted during which time the composition was applied to the test site twice daily (excluding the weekends). Dansyl chloride stain was then administered to both the treated and untreated sites. This was followed by a 3-5 week period during which the twice daily application of the present composition to the treated site was continued.

The results of this study indicated that the test product enhanced epidermal cell renewal by approximately 25% over no treatment.

Example 15

A test of the retinol containing composition of Example 14 to induce retinoid benefits to the skin was conducted. The composition (Example 14) was applied to the upper inner arm of ten women aged 25-45 twice daily for a seven week period. Superficial shave biopsies of the epidermis were taken from the treated site and from an adjacent untreated control area. Histological observations for retinoid effects were made by a leading clinical dermatologist.

The results of this study indicated that retinoid effects were observed in 30% of the panelists. These effects included improvement in vertical orientation and cellular stacking in the epidermis and slight increase in the size of the cells. Further, 20% of the panelists demonstrated an increase in viable epidermis thickness.

Example 16

Rhino mouse skin studies were conducted to determine the effectiveness of retinol in normalizing epidermal skin structures. Rhino mice normally have wrinkled, sagging skin. The rhino mouse test is used as a model for showing the effects of compositions on the epidermis. In the test each set of seven mice was treated for five days/week for six consecutive weeks. Five sets of mice were treated. The treating agents were similar to the vehicles of formulation J in Example 9 without the retinol blend and that vehicle with the retinol blend, with the retinol content at concentrations of 0.005, 0.01, and 0.03 weight percent. Specifically, the four silicone emulsions contained:

|  | I | II | III | IV |
|---|---|---|---|---|
| Demineralized Water | 32.08388 | 32.08388 | 32.08388 | 32.08388 |
| Gly Acrylic Polymer (Lubragel)® | 20.00020 | 20.00020 | 20.00020 | 20.00020. |
| Cyclomethicone-Tetramer | 14.44968 | 14.45852 | 14.41955 | 14.46924 |
| Cyclometh/Dimeth Copolyol 90/10 | 10.00000 | 10.00000 | 10.00000 | 10.00000 |
| Cyclomethicone-Pentamer | 3.50064 | 3.50064 | 3.50064 | 3.50064 |
| Pentrolatum | 5.00000 | 5.00000 | 5.00000 | 5.00000 |
| Ethylhexyl Palmitate | 5.00000 | 5.00000 | 5.00000 | 5.00000 |
| Alcohol SD 40B Anhydrous | 4.00000 | 4.00000 | 4.00000 | 4.00000 |
| Phenyl Benzimidazole-5-Sulf Acid | 2.00000 | 2.00000 | 2.00000 | 2.00000 |
| Triethanolamine 99% | 1.10000 | 1.10000 | 1.10000 | 1.10000 |
| Stearyl ETO (20M) Alcohol | 1.00000 | 1.00000 | 1.00000 | 1.00000 |
| Sodium Chloride | 1.00000 | 1.00000 | 1.00000 | 1.00000 |
| Hyaluronic Acid Sod. Salt | .03960 | .03960 | .03960 | .03960 |
| Methylparaben | .40000 | .40000 | .40000 | .40000 |
| Butylated Hydroxytoluene | .22000 | .22000 | .22000 | .22000 |
| POE (20M) Sorbitan Monolaurate | .03000 | ------ | ------ | ------ |
| Germall® | .17600 | .17600 | .17600 | .17600 |
| Retinoid Blend (Example I) | ------ | .02088 | .06013 | .01044 |
| Beta-Carotene | ------ | .00028 | ------ | ------ |

Visual observation of the skin condition during the period of treatment showed significant diminution of the characteristic epidermal utriculi and an increase in viable epidermal thickness in the sets of mice treated with the present formulations containing retinol when compared with the sets of mice either untreated or treated with the vehicle alone.

Example 17

A test of the effectiveness of the formulation in Example 14 to visibly reduce tiny dry facial lines in the crowsfeet areas was conducted to assess its moisturizing ability. Twenty-eight women who were prescreened for lines in the crowsfeet area participated in the study. Panelists applied the test product twice daily for at lease two weeks to only one side of the face and left the other side untreated to serve as the control site. After application of the composition of Example 14, a trained evaluator, who had no knowledge of the treatment side, rated the panelist's lines on both sides of the face. The results of this study indicated that there was a statistically significant visual improvement in the number and depth of fine dry lines after just seven days application of the test product.

Example 18

A test of the ability of the composition of Example 14 to reduce skin dryness was conducted. Twelve panelists who demonstrated skin dryness upon repeated soap washing of the hands were selected to participate in this study. Initially, the panelists induced a condition of dryness by washing their hands with bar soap. The test product was applied daily to one hand while the other was left untreated to serve as a control side. Each hand was rated randomly by two trained evaluators who had no knowledge of which hand had been treated. The evaluators used a stereomicroscope to assist them with their ratings. The results of this study revealed that effective moisturization benefits were demonstrated throughout treatment by the composition of Example 14. In addition, these benefits persisted for twenty-four hours after the final treatment indicating that the test product provides effective long-lasting moisturization.

It will thus be appreciated that moisturizing efficacy can be achieved with the compositions of the present invention containing the retinol, thereby precluding the need for a separate moisturizer. Therefore, preferred compositions of the invention can be formulated to include moisturizing components that are compatible with the alcohol lotion or silicone emulsion to a level of up to 35% by weight of the final formulation.

**Claims**

1. A composition comprising retinol, a volatile silicone and a mutual solvent for retinol and the volatile silicone,

2. A composition as claimed in claim 1 wherein the volatile silicone is a cyclomethicone.

3. A composition as claimed in claim 1 or claim 2 wherein the mutual solvent is ethanol.

4. A composition as claimed in any of claims 1 to 3 wherein the weight ratio of silicone to mutual solvent is about 6:4.5.

5. A composition as claimed in any of claims 1 to 4 containing 0.005 to 1.0 weight percent retinol.

6. A composition as claimed in claim 5 containing 0.01 to 0.50 weight percent retinol.

7. A composition as claimed in any of claims 1 to 6 further comprising one or more emollients, moisturizing agents, thickening agents, UV absorbers, sunscreens, antioxidants, preservatives, emulsifiers and/or emulsion stabilisers.

8. A composition as claimed in any of claims 1 to 6 comprising:
   retinol;
   at least 20 weight percent of a cyclomethicone;
   25 to 60 weight percent ethanol; and
   0 to 15 weight percent emollient.

9. A process for the preparation of a composition as claimed in claim 5 wherein a composition formulatd with a higher level of retinol content is diluted with a cosmetically acceptable vehicle in an amount sufficient to reduce the level of retinol to between 0.005 and 1.0 weight percent.

10. A process as claimed in claim 9 wherein the vehicle is a water in oil emulsion.

11. A process as claimed in claim 10 wherein the weight ratio of the oil phase to the water phase is 1:2.

12. A process as claimed in any of claims 9 to 11 wherein the final composition comprises 0.1 to 1.0 weight percent retinol and 15 to 35 weight percent cyclomethicone.

13. A method of reducing skin wrinkles comprising topically applying to the skin a retinol composition as claimed in claims 5 or 6 or prepared as claimed in any of claims 9 to 12.

14. A method as claimed in claim 13 wherein a skin moisturizer is applied to the skin at substantially the same time as the retinol composition is applied or a skin moisturizing ingredient is included in the retinol composition.

**Revendications**

1. Composition comprenant du rétinol, une silicone volatile, et un solvant du rétinol et de la silicone volatile.

2. Composition telle que revendiquée dans la revendication 1, dans laquelle la silicone volatile est une cyclométhicone.

3. Composition telle que revendiquée dans la revendication 1 ou la revendication 2, dans laquelle le solvant est l'éthanol.

4. Composition telle que revendiquée dans l'une des revendications 1 à 3, dans laquelle le rapport pondéral de la silicone au solvant est d'environ 6 : 4,5.

5. Composition telle que revendiquée dans l'une des revendications 1 à 4 contenant 0,005 à 1,0 pourcent en poids de rétinol.

6. Composition telle que revendiquée dans la revendication 5 contenant 0,01 à 0,5 pourcent en poids de rétinol.

7. Composition telle que revendiquée dans l'une des revendications 1 à 6 comprenant également un ou plusieurs émollients, agents d'humidification, agents épaississants, absorbants d'UV, écrans solaires, antioxydants, conservateurs, émulsifiants et/ou stabilisateurs d'émulsion.

8. Composition telle que revendiquée dans l'une des revendications 1 à 6 comprenant :
   - du rétinol ;
   - au moins 20 % en poids d'une cyclométhicone ;
   - 25 % à 60 % en poids d'éthanol ; et
   - 0 à 15 % en poids d'un émollient.

9. Procédé pour la préparation d'une composition telle que revendiquée dans la revendication 5, dans lequel une composition formulée de teneur en rétinol plus élevée est diluée avant un véhicule acceptable pour des applications cosmétiques, dans une quantité suffisante pour réduire la teneur en rétinol à une valeur comprise entre 0,005 à 1,0 pourcent en poids.

10. Procédé tel que revendiqué dans la revendication 9 dans lequel le véhicule est une émulsion eau dans huile.

11. Procédé tel que revendiqué dans la revendication 10, dans lequel le rapport pondéral de la phase huileuse à la phase aqueuse est de 1 : 2.

12. Procédé tel que revendiqué dans l'une des revendications 9 à 11, dans lequel la composition finale comprend de 0,1 à 1,0 pourcent en poids de rétinol et de 15 à 35 pourcent en poids de cyclométhicone.

13. Procédé de réduction des rides de la peau, comprenant l'application topique sur la peau d'une composition de rétinol telle que revendiquée dans les revendications 5 ou 6 ou préparée de façon telle que revendiquée dans l'une des revendications 9 à 12.

14. Procédé tel que revendiqué dans la revendication 13, dans lequel un humidificateur de la peau est appliqué sur la peau substantiellement en même temps que la composition de rétinol est appliquée, ou un ingrédient d'humidification de la peau est inclus dans la composition de rétinol.

**Patentansprüche**

1. Zusammensetzung, enthaltend Retinol, ein flüchtiges Silicon und ein gemeinsames Lösungsmittel für Retinol und das flüchtige Silicon.

2. Zusammensetzung gemäß Anspruch 1, worin das flüchtige Silicon ein Cyclomethicon ist.

3. Zusammensetzung gemäß Anspruch 1 oder 2, worin das gemeinsame Lösungsmittel Ethanol ist.

4. Zusammensetzung gemäß einem der Ansprüche 1 bis 3, worin das Gewichtsverhältnis von Silicon zu dem gemeinsamen Lösungsmittel etwa 6:4,5 beträgt.

5. Zusammensetzung gemäß einem der Ansprüche 1 bis 4, enthaltend 0,005 bis 1,0 Gew.% Retinol.

6. Zusammensetzung gemäß Anspruch 5, enthaltend 0,01 bis 0,50 Gew.% Retinol.

7. Zusammensetzung gemäß einem der Ansprüche 1 bis 6, weiterhin enthaltend ein oder mehrere Erweichungsmittel, Befeuchtungsmittel, Verdickungsmittel, UV-Absorber, Sonnenfilter, Antioxidantien, Konservierungsmittel, Emulgiermittel und/oder Emulsionsstabilisatoren.

8. Zusammensetzung gemäß einem der Ansprüche 1 bis 6, enthaltend
   Retinol;

zumindest 20 Gew.% eines Cyclomethicons;
25 bis 60 Gew.% Ethanol; und
0 bis 15 Gew.% Erweichungsmittel.

9.  Verfahren zur Herstellung einer Zusammensetzung gemäß Anspruch 5, worin eine Zusammensetzung mit einem höheren Wert des Retinolgehalts mit einem kosmetisch verträglichen Träger in einer ausreichenden Menge verdünnt wird, um den Wert des Retinols auf einen Wert zwischen 0,005 und 1,0 Gew.% zu reduzieren.

10. Verfahren gemäß Anspruch 9, worin der Träger eine Wasser-in-Öl-Emulsion ist.

11. Verfahren gemäß Anspruch 10, worin das Gewichtsverhältnis der Ölphase zu der Wasserphase 1:2 beträgt.

12. Verfahren gemäß einem der Ansprüche 9 bis 11, worin die endgültige Zusammensetzung 0,1 bis 1,0 Gew.% Retinol und 15 bis 35 Gew.% Cyclomethicon umfaßt.

13. Verfahren zur Verminderung von Hautfalten, umfassend das topische Auftragen auf die Haut einer Retinol-Zusammensetzung, wie in den Ansprüchen 5 oder 6 beansprucht oder wie in einem der Ansprüche 9 bis 12 hergestellt.

14. Verfahren gemäß Anspruch 13, worin ein Hautbefeuchter im wesentlichen gleichzeitig mit dem Auftragen der Retinol-Zusammensetzung auf die Haut aufgetragen wird oder ein Hautbefeuchtungsbestandteil in die Retinol-Zusammensetzung eingeschlossen wird.